# EUROPEAN PATENT SPECIFICATION

(11) **EP 1 845 902 B1**
(45) Date of publication and mention of the grant of the patent: **10.12.2008**
(21) Application number: 05854588.0
(22) Date of filing: 16.12.2005
(51) Int. Cl.: A61F 2/44

(54) **ARTIFICAL SPINAL DISC**
KÜNSTLICHE BANDSCHEIBE
DISQUE VERTEBRAL ARTIFICIEL

(30) Priority: 17.12.2004 US 15104
(43) Date of publication of application: 24.10.2007
(73) Proprietor: O'Savage Biomechanics, Inc., Cleveland OH 44141 (US)
(72) Inventor: PAXSON, Robert, D., Lakeland, TN 38002 (US); NILSSON, Carl, M., Cleveland Heights, OH 44121 (US); SAVAGE, Daniel, S., Brecksville, OH 44141 (US)
(74) Representative: Hilleringmann, Jochen
(86) International application number: PCT/US2005/045908
(87) International publication number: WO 2006/066198

(56) References cited:
- WO-A-00/23015
- WO-A-20/04019828
- WO-A-20/04026186
- DE-U1- 20 310 432
- DE-U1- 20 313 183
- US-A1- 2004 176 772

## Description

### FIELD OF THE INVENTION

The present invention relates generally to prostheses for treating spinal pathologies, and more specifically to artificial disc replacements and components thereof that improve the fit and functionality of such replacements.

### BACKGROUND OF THE INVENTION

Back pain is a common ailment. In many cases, the pain severely limits a person's functional ability and quality of life. A variety of spinal pathologies can lead to back pain. In the treatment of diseases, injuries or malformations affecting spinal motion segments, and especially those affecting disc tissue, it has long been known to remove some or all of a degenerated, ruptured or otherwise failing disc. In cases involving intervertebral disc tissue that has been removed or is otherwise absent from a spinal motion segment, corrective measures are taken to insure the proper spacing of the vertebrae formerly separated by the removed disc tissue.

In some instances, the two adjacent vertebrae are fused together using transplanted bone tissue, an artificial fusion component, or other compositions or devices. Spinal fusion procedures, however, have raised concerns in the medical community that the biomechanical rigidity of intervertebral fusion may predispose neighboring spinal motion segments to rapid deterioration. More specifically, unlike a natural intervertebral disc, spinal fusion prevents the fused vertebrae from pivoting and rotating with respect to one another. Such lack of mobility tends to increase stresses on adjacent spinal motion segments. Additionally, several conditions may develop within adjacent spinal motion segments, including disc degeneration, disc herniation, instability, spinal stenosis, spondylosis and facet joint arthritis. Consequently, many patients may require additional disc removal and/or additional surgical procedures as a result of spinal fusion. Alternatives to spinal fusion are therefore desirable.

Several different types of artificial disc replacement devices have been proposed for preventing the collapse of the intervertebral space between adjacent vertebrae while maintaining a certain degree of stability and range of pivotal and rotational motion therebetween. Such devices typically include two or more articular elements that are attached to respective upper and lower vertebrae. The articular elements are anchored to the upper and lower vertebrae by a number of methods, including the use of bone screws that pass through corresponding openings in each of the elements and thread into vertebral bone, and/or by the inclusion of spikes or teeth that penetrate the vertebral endplates to inhibit migration or expulsion of the device. The articular elements are typically configured to allow the elements, and correspondingly the adjacent vertebrae, to pivot and/or rotate relative to one another.

Artificial disc implants have several advantages over spinal fusion. The most important advantage of an artificial disc implant is the preservation of spinal motion. An artificial disc replacement, however, also allows motion through the facet joints. Motion across arthritic facet joints could lead to pain following artificial disc replacement. Some surgeons believe patients with degenerative disease and arthritis of the facet joints are not candidates for artificial disc replacements.

Current artificial disc implant designs do not attempt to limit the pressure across the facet joints or facet joint motion. Indeed, prior art artificial disc implants generally do not restrict motion. For example, some artificial disc implant designs place bags of hydrogel into the disc space. Hydrogel bags do not limit motion in any direction. In fact, bags filled with hydrogels may not provide distraction across the disc space. Current art artificial disc implant designs with metal plates and polyethylene spacers may restrict translation but they do not limit the other motions mentioned above.

Although artificial disc replacement permits more motion than does spinal fusion, there is a general need in the industry to provide an improved artificial disc implant that allows a patient to achieve more natural flexion, rotation, extension, and bending following artificial disc replacement surgery, while minimizing the variation of contact pressure on other aspects of the vertebrae. The present invention satisfies this need and provides other benefits and advantages in a novel and unobvious

An artificial disc implant according to the preamble of claim 1 is disclosed in WO-A-2004/026186.

### BRIEF SUMMARY OF THE INVENTION

According to the present invention, there is provided an artificial disc implant comprising: a superior implant configured for placement on a superior vertebra; an inferior implant configured for placement on an inferior vertebra; and an articulating interface between the superior vertebra and the inferior vertebra, the articulating interface being generally saddle-shaped and ramped, wherein the articulating interface generally progresses away from the superior vertebra and toward the inferior vertebra as the interface progresses from a first side of the artificial disc implant to an opposing side of the artificial disc implant.

### BRIEF DESCRIPTION OF THE DRAWINGS

Figure 1 is a lateral elevation view of human cervical vertebrae;
Figure 2 is lateral view of human cervical vertebrae illustrating a coupled lateral bending and axial rotation axis;
Figure 3 is an anterior view of human cervical vertebrae illustrating an axial rotation axis and a flexion/extension axis;
Figure 4 is an exploded perspective view of an artificial disc implant of the present invention;
Figure 5 illustrates an artificial disc implant of the present invention in conjunction with human cervical vertebrae in a lateral elevation view;
Figure 6 illustrates an embodiment of the artificial disc implant of the present invention that is specifically configured for fixation to human cervical vertebrae using fixation screws;
Figure 7 illustrates an embodiment of the artificial disc implant of the present invention that allows for axial rotation of a spacer with respect to an inferior implant; and
Figure 8 illustrates an embodiment of the artificial disc implant of the present invention that allows for axial rotation and generally in-plane motion of a spacer with respect to an inferior implant.

### DETAILED DESCRIPTION OF THE INVENTION

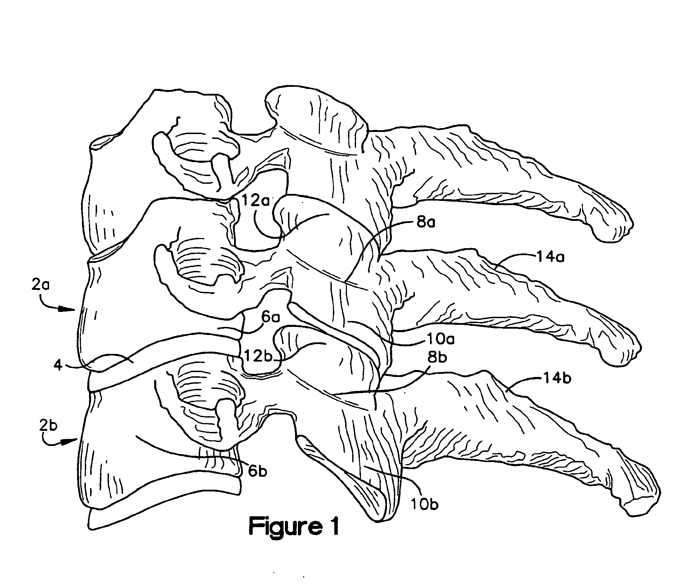

Turning now to Figure 1, normal human cervical vertebrae are illustrated. A superior vertebra 2a is formed above the inferior vertebra 2b. For example, in the C3-C4 facet joint, the superior vertebra 2a is the C3 vertebra and the inferior vertebra 2b is the C4 vertebra. Between the vertebrae 2 is an intervertebral disc 4. It will be understood by those skilled in the art that while the cervical vertebrae 2 vary somewhat according to location, they share many features common to most vertebrae.

Each vertebra 2 includes a vertebral body 6. Connected to the vertebral body 6 is a lateral mass 8. Two inferior articular processes extend downward from the junction of the laminae 14 and the transverse processes. The inferior articular processes a each have a natural bony structure known as an inferior articular facet 10, which faces downward. Similarly, a superior articular facet 12 faces upward from the junction of the lateral mass 8 and the pedicle. When adjacent vertebrae 12 are aligned, the superior articular facet 12 and inferior articular facet 10 interlock. The intervertebral disc 4 between each pair of vertebrae 2 permits gliding movement between vertebrae 2. Thus, the structure and alignment of the vertebrae 2 permit a range of movement of the vertebrae 2 relative to each other.

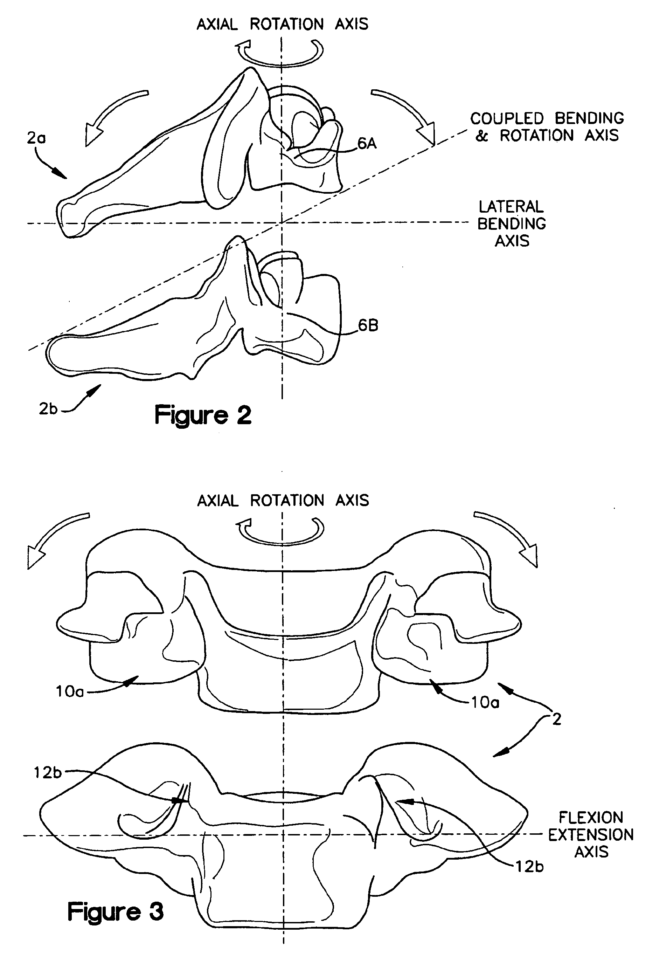

Turning next to Figures 2 and 3, an anterior view of human cervical vertebrae showing an axial rotation axis, a lateral bending axis and a flexion/extension axis is illustrated. The three axes, axial rotation, lateral bending and flexion/extension are essentially orthogonal in nature. Flexion is the anterior movement of the upper vertebra 2a, extension is the posterior movement of the upper vertebra 2a. The flexion/extension axis is generally oriented to go from side to side of the vertebra, roughly parallel to the upper endplate of the inferior vertebra 2b. The flexion/extension axis is located in various locations as you move up and down the spine, but generally is perpendicular to the sagittal plane and located on or below the endplate of the inferior vertebra 2b and between the posterior edge and the mid section of the vertebra 2b. The total range of motion about the flexion/extension axis is approximately 30 degrees per level. Rotation about the flexion/extension axis is independent of motions along the other axes.

The lateral bending axis is generally horizontal in nature and passes from the anterior to the posterior between the vertebrae 2a and 2b. The range of motion of bending about the lateral bending axis is approximately 10 degrees per side, for a total of approximately 20 degrees.

The axial rotation axis is generally vertical in nature and passes through both the superior vertebra 2a and inferior vertebrae 2b. The axial rotation axis passes through the vertebral bodies 6a and 6b in the posterior half of the vertebral bodies 6a and 6b, but anterior to the spinal cord. Generally in the cervical spine, each disc level may see up to approximately 10 degrees of axial rotation per side, to a combined approximately 20 degrees of rotational motion.

Though the lateral bending and axial rotation axes each permit about 10 degrees of rotation in each direction, rotation along the lateral bending axis and rotation along the axial rotation axis cannot occur independently of one another without causing high stress in the intervertebral disc 4 and articular facets 10a and 12b. In other words, no lateral bending can occur in the cervical spine without axial rotation. Conversely, no axial rotation can occur with lateral bending. Thus, the lateral bending and axial rotation axes are coupled in the cervical spine.

These coupled motions can be described as rotation about a single coupled axis-the coupled axial rotation and lateral bending axis. The location and trajectory of this axis is determined by the facet joint and the uncovertebral joints. For example, a ratio of 1:1 for lateral bending to rotation may yield a coupled motion axis that is approximately 45 degrees above the horizontal. This angle relates inversely to the ratio of lateral bending motion to rotational motion. Thus, as the ratio increases, the axis will be closer to the horizontal plane. Ultimately for a device to accurately recreate anatomical motion in the cervical spine, this coupled axis is located in the sagittal plane, above the cervical disc space and is angled upward from posterior to anterior. The axis of rotation for the coupled axis may vary for each disc level and among individuals.

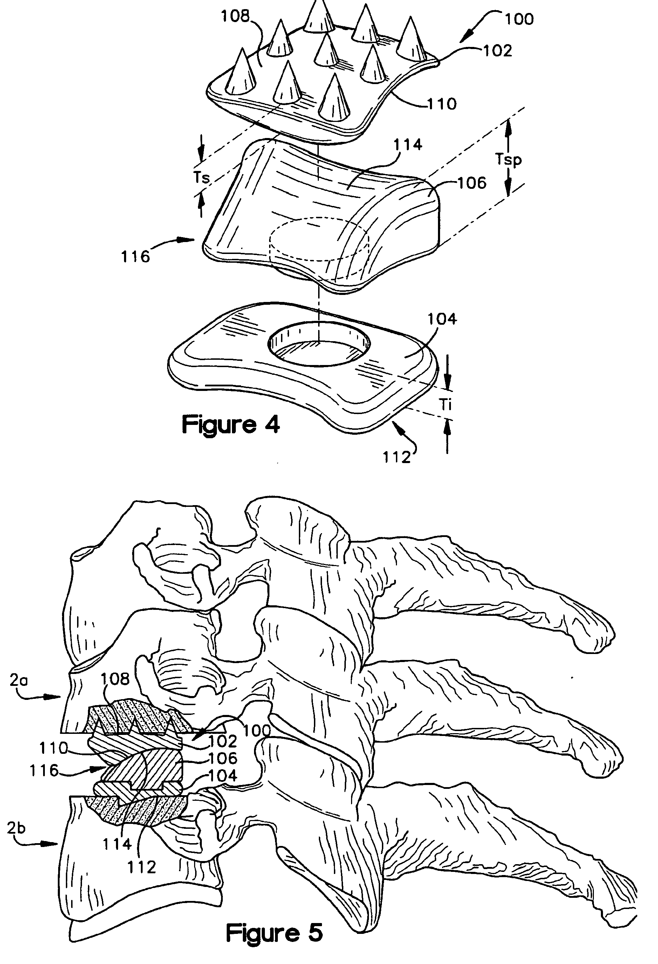

Turning next to Figures 4 and 5, Figure 4 illustrates an exploded perspective view of an embodiment of artificial disc implant according to the present invention and Figure 5 illustrates the artificial disc implant of Figure 4 in use as a replacement for a cervical intervertebral disc 4. The articulating interface 116 can generally be described as a ramped saddle shaped surface. This saddle shape is defined by two rotational axes which correspond with the flexion/extension axis and the coupled lateral bending and axial rotation axis.

The coupled lateral bending and axial rotation axis is located within the mid-sagittal plane and is generally perpendicular to the plane of the facet joint. The normal distance of the coupled motion axis to the flexion/extension axis is dependent on the geometry of the uncovertebral joints. The normal distance can be determined by viewing a cross-section of the vertebrae in a plane parallel to the facet joint, or perpendicular to the resulting coupled motion axis. Preferably, this plane also passes through the flexion/extension axis. The uncovertebral joint appears in this cross-section as angled surfaces/lines on the left and right side of an endplate, which appears as a middle flat portion. A circle can then be fitted such that it is tangent to the two angled surfaces/lines. The center of the circle then indicates the location of the coupled lateral bending and axial rotation axis. Once the location of the coupled lateral bending and axial rotation axis is determined, the distance to the flexion/extension axis can then be calculated.

Both axes can then be used to define the contacting surfaces for both inferior and superior surfaces. To define the articulating surface 114 of the inferior implant 104 or spacer 106, a circle similar to the circle used to locate the coupled lateral bending and axial rotation axis is placed within that same plane used to locate the lateral bending and axial rotation axis. The circle is then rotated around the flexion/extension axis. The resulting surface that such a rotation would cut out of a block of material is the articulating surface 114. For example, if the rotated circle were to create a solid object, it would resemble a donut.

The articulating surface 110 of the superior implant 102 can be defined by creating a circle in the midsagittal plane with a radius corresponding to the bending radius during flexion/extension with a center point at the flexion/extension axis. This circle is then rotated around the coupled motion axis. The resulting surface that such a rotation would cut out of a block of material is the articulating surface 110 of the superior implant 104. Again, this would resemble a donut if the rotated circle were to create a solid object.

Additional machining operations may then be performed, for example, to remove excess material or to round corners. The additional machining processes, however, preferably do not alter the articulating surface created.

Using this technique, an implant 102 may be designed such that the implant mimics the natural movement patters of the vertebrae so that stresses on the joint and on adjacent disc levels are minimized.

It will be understood by those skilled in the art that a variety of other manufacturing processes may be used to generate the inventive implant.

In addition, the articulating interface 116 may be also designed such that it has one or more neutral zone. Neutral zones may be used in the design of the artificial disc implant 100 to allow for anatomical variations and for inexact placement of the implant 100 or components thereof during surgery by the surgeon. Neutral zones may also be useful to permit variation in the anatomy and the location of rotational axes and to permit inexact placement of superior and inferior implants with respect to each other by the surgeon during surgery while supporting the desired natural movement pattern.

For example, a neutral zone may be located on the articulating surface of either the spacer 106 or the inferior implant 104. Such a neutral zone allows for lateral shifts between superior implant 102 and inferior implant 104. The neutral zone may be, for example, a flat region that is created when the articulating surface is cut in half along the mid-sagittal plane. The resulting two halves may be separated so that a constant cross-section over a specified width is defined between them. This width, or neutral zone, may range from about 0 mm to about 5 mm. In a presently preferred embodiment, the neutral zone is about 3 mm.

A neutral zone may also be located on the articulating surface of the superior implant 104. Such a neutral zone allows for anterior/posterior shifts between the superior implant 102 and inferior implant 104 of up to the width of the neutral zone. This neutral zone may be defined by cutting the superior implant in half by an anterior/posterior plane that falls through the flexion/extension axis. The resulting two halves may be separated so that a constant cross-section over a specified width is defined between them. This width may range from about 0 mm to about 5 mm. In a presently preferred embodiment, the neutral zone is about 3 mm.

As shown in Figures 4 and 5, the implant 100 includes a superior implant 102 configured for placement on a superior vertebra 2a and an inferior implant 104 configured for placement on an inferior vertebra 2b. The implant also includes an articulating interface 116 between the superior vertebra and the inferior vertebra where the articulating interface is configured such that movement between the superior and inferior implants 102 and 104 about an axial rotation axis causes movement between the superior and inferior implants 102 and 104 about a lateral bending axis.

The physical shape and configuration of various embodiments of the implant 100 are described as follows. Generally, the artificial disc implant 100 includes a superior implant 102 that is configured for placement on a superior vertebral body 6a. The artificial disc implant 100 also includes an inferior implant 104 that is configured for placement on an inferior vertebral body 6b. An articulating interface 116 is formed by an articulating surface 110 of the superior implant 102 and by a second articulating surface, which may be an articulating surface 114 of a spacer or an articulating surface of an inferior implant, depending whether the spacer 106 and the inferior implant 104 are combined into a single component.

The articulating interface 116 is generally saddle-shaped and ramped such that the articulating interface 116 generally progresses toward the superior vertebral body and away from the inferior vertebral body as the articular interface 116 progresses from the anterior to the posterior of the artificial disc implant 100.

It will be understood by those skilled in the art that the artificial disc implant of the present invention may have a superior implant having an articulating surface, an inferior implant, and a spacer having an articulating surface. In this embodiment, the spacer is fixed or attached to the inferior implant. Because the presently preferred embodiment includes a superior implant, an inferior implant and a spacer, all figures are directed toward variations of artificial disc implants having a superior implant, an inferior implant and a spacer.

In addition, the artificial disc implant of the present invention may also be comprised of a superior implant having an articulating surface and an inferior implant having an articulating surface. In other words, the inferior implant and the spacer could be combined into a single component of the artificial disc implant.

The superior implant 102 comprises a fixation surface 108 and an articulating surface 110. The superior implant 102 is configured for placement on a superior vertebral body 6a. The superior implant 102 may be fixed to the superior vertebral body 6a using cemented fixation techniques, cementless fixation techniques, or a combination thereof. In an exemplary embodiment, the superior implant 102 has a fixation surface 108 that is configured for placement on a specifically prepared superior vertebral body 6a. The articulating surface 110 is configured to interact with an articulating surface 114 of a spacer 106 (or of an inferior implant 104) to form an articulating interface 116.

The superior implant 102 preferably has a fixation mechanism for fixing the superior implant 102 to the superior vertebral body 6a. The fixation mechanism may be any fixation mechanism known in the art, such as: one or more pegs, one or more fins, one or more pips, one or more spikes, one or more pins ridges or grooves, one or more screws, and the like. In one exemplary embodiment, the fixation surface 108 of the superior implant 102 is configured to interact only with a specifically prepared surface of the superior vertebral body 6a. In another embodiment, the superior implant 102 may have a fixation mechanism that is configured to interact with just the side of the superior vertebral body 6a. The fixation surface 108 of the superior implant 102 may be generally flat, generally curved or generally dome-shaped for improved interaction with the superior vertebral body 6a. In one embodiment, the fixation surface 108 is generally curved.

The fixation surface 108 may also have a porous coating; a porous onlay material; a biologic or biocompatible coating; a surface treatment, such as to facilitate bone ingrowth or cement fixation; and combinations thereof. For example, the fixation surface 108 may have a porous surface that is beaded, threaded, textured, or the like to facilitate bone ingrowth. Further, the fixation surface 108 may have a hydroxyapatite coating or may be plasma-sprayed. In addition to the examples listed, any known method of improving fixation of biologic implants may be used to improve the interaction of the superior implant 102 and the superior vertebral body 6a.

The articulating surface 110 of the superior implant 102 is generally configured to articulate or interact with the articulating surface 114 of a spacer 106 or a combination spacer/inferior implant. The articulating surface 110 is generally saddle-shaped and ramped from the posterior of the superior implant 102 to the anterior of the implant 100. In other words, the articulating surface 110 generally progresses away from the superior vertebra as it progresses from the posterior to the anterior of the implant 100. The apex of the ramp of the articulating surface 110 in one embodiment is near the anterior end of the implant, but not at the anterior end of the implant.

In addition, the superior implant 102 in the presently preferred embodiment is generally convex. In other words, the superior implant 102 generally has a thicker depth at points along the midline 118 progressing from the anterior to the posterior than at the sides at the same distance along the midline 118. It should be noted, however, that the articulating surface 110 of the superior implant 102 may also be generally concave such that it is thinner in depth at points along the midline 118 progressing from the anterior to the posterior than it is at the sides at the same distance along the midline.

The superior implant 102 may be composed of any material known in the art for articulating medical implants. Such materials include, but are not limited to, cobalt-chromium alloys, ceramics (alumina ceramic, zirconia ceramic, yttria zirconia ceramic, etc.), titanium, ultra high molecular weight polyethylene (UHMWPE), pyrolytic carbon, titanium/aluminum/vanadium (Ti/Al/V) alloys, Tantalum, carbon composite materials and combinations thereof. For example, the superior implant 102 may be generally composed of a ceramic material or a cobalt-chromium alloy. Some materials are more appropriate for articulating surfaces and some more appropriate for fixation surfaces, but any materials known in the art for use with articulating and fixation surfaces can be used in the present invention. Such materials are commonly used in joint arthroplasties and the like.

The superior implant 102 may range from about 1 mm thick to about 5 mm thick at the anterior of the superior implant 102 and from about 1 mm to about 5 mm thick at the posterior of the superior implant 102. In an exemplary embodiment, the thickness (Tₛ) of the superior implant 102 ranges from about 1 mm thick to about 2 mm thick at the anterior of the superior implant 102 and from about 1 mm to about 2 mm at the posterior of the superior implant 102. Also, the mid portion of the superior implant may range from about 0.5 mm to about 2 mm.

The inferior implant 104 comprises a fixation surface 112. The inferior implant 104 is configured for placement on an inferior vertebral body 6b and is configured to interact with the spacer 106. Again, it should be understood that the spacer 106 and the inferior implant 104 may be combined into a single component of the intervertebral disc implant 100. The inferior implant 104 may be fixed to the inferior vertebral body 6b using cemented and/or cementless fixation techniques. In an exemplary embodiment, the inferior implant 104 has a fixation surface 112 that is configured for placement on a specifically prepared inferior vertebral body 6b.

The inferior implant 104 preferably has a fixation mechanism for fixing the inferior implant 104 to the inferior vertebral body 6b. The fixation mechanism may be any fixation mechanism known in the art, such as: one or more pegs, one or more fins, one or more pips, one or more spikes, one or more pins ridges or grooves, one or more screws, and the like. In one exemplary embodiment, the fixation surface 112 of the inferior implant 104 is configured to interact only with a specifically prepared surface of the superior vertebral body 6b. In another embodiment, the inferior implant 104 may have a fixation mechanism that is configured to interact with just the side of the inferior vertebral body 6b. The fixation surface 112 of the inferior implant 104 may be generally flat, generally curved or generally dome-shaped for improved interaction with the inferior vertebral body 6b. In one embodiment, the fixation surface 112 is generally flat.

The fixation surface 112 may also have a porous coating; a porous onlay material; a biologic or biocompatible coating; a surface treatment, such as to facilitate bone ingrowth or cement fixation; and combinations thereof. For example, the fixation surface 112 may have a porous surface that is beaded, threaded, textured, or the like to facilitate bone ingrowth. Further, the fixation surface 112 may have a hydroxyapatite coating or may be plasma-sprayed. In addition to the examples listed, any known method of improving fixation of biologic implants may be used to improve the interaction of the inferior implant 104 and the inferior vertebral body 6b.

The inferior implant 104 may be composed of any material known in the art for articulating medical implants. Such materials include, but are not limited to, cobalt-chromium alloys, ceramics (alumina ceramic, zirconia ceramic, yttria zirconia ceramic, etc.), titanium, UHMWPE, pyrolytic carbon, Ti/AIN alloys, Tantalum, Carbon composite materials and combinations thereof. For example, the inferior implant 104 may be generally composed of cobalt-chromium or titanium and may have a titanium nitride coating. If the inferior implant 104 and the spacer 106 are combined into a single inferior implant, the inferior implant may utilize any bearing material that is appropriate as an articulating counterface with the superior implant's articulating surface. For example, if the superior articulating surface is cobalt-chromium alloy, UHMWPE would be an appropriate bearing counterface.

When a spacer 106 is used, the inferior implant 104 may range from about 0.5 mm thick to about 5 mm thick. In an exemplary embodiment, the thickness (Tᵢ) of the inferior implant 104 ranges from 0.5 mm thick to about 2 mm.

When the spacer is combined with the inferior implant 104, the inferior implant 104 may range from about 0.5 mm thick to about 5 mm thick at the anterior of the inferior implant 104 and from about 0.5 mm to about 5 mm thick at the posterior of the superior implant 104, while the mid section may range from about 1 mm to about 10 mm thick. In an exemplary embodiment, the thickness ranges from about 0.5 mm thick to about 2 mm thick at the anterior of the inferior implant 104 and from about 0.5 mm to about 2 mm at the posterior of the inferior implant 104.

The spacer 106 is preferably configured to interact with the inferior implant 104 and may be capable of rotation and/or generally planar motion with respect to the inferior implant 104. The interaction between the spacer 106 and the inferior implant 104 may vary. For example, in one embodiment, a tapered pin system can be used to help prevent the spacer 106 from posterior to anterior movement once correctly positioned. Various systems and designs known in the art can be used to achieve the desired interaction between the spacer 106 and the inferior implant 104.

The spacer 106 includes an articulating surface 114 that is configured to articulate with the articulating surface 110 of the superior implant 102. The spacer 106 may range from about 0.5 mm thick to about 5.5 mm thick at the anterior of the spacer 106 and from about 0.5 mm to about 5.5 mm thick at the posterior of the spacer 106, while the mid section may range from about 2 mm to about 8 mm. In an exemplary embodiment, the thickness (Tₛₚ) of the spacer 106 ranges from about 0.5 mm thick to about 4.5 mm thick at the anterior of the spacer 106 and from about 0.5 mm to about 4.5 mm at the posterior of the spacer 106, while the mid section may range from about 3 mm to about 6 mm.

Whether the spacer 106 and the inferior implant 104 are combined or exist as separate components, the articulating surface (shown as the articulating surface 114 of the spacer) is configured to articulate with the articulating surface 110 of the superior implant. The articulating surface 114 is generally saddle-shaped and ramped from the anterior of the artificial disc implant 100 to the posterior of the artificial disc implant 100. In other words, the articulating surface 114 generally progresses toward from the superior vertebra as it progresses from the anterior to the posterior of the artificial disc implant 100. The apex of the ramp of the articulating surface 114 in one embodiment is near the posterior end of the implant, but not at the posterior end of the implant. While the articulating surfaces 110 and 114 of the preferred embodiment ramp as described above, it will be understood that the articulating surfaces 110 and 114 may ramp in other directions, such as laterally, in the opposite direction as described above, or in any direction there between.

In addition, the articulating surface 114 in the presently preferred embodiment is generally concave. In other words, the spacer 106 (or inferior implant/spacer combination) has a thinner depth at points along the midline 120 progressing from the anterior to the posterior than at the sides at the same distance along the midline 120. It should be noted, however, that the articulating surface 114 may also be generally convex such that it is generally thicker in depth at points along the midline 120 progressing from the anterior to the posterior than it is at the sides at the same distance along the midline 120.

The artificial disc implant 100 may also be configured to allow for the separation of lateral bending and axial rotation. In other words, the superior implant 102 may be free to rotate freely with respect to the inferior implant 104. Further, the artificial disc implant 100 may permit generally in-plane movement between two or more of the components of the artificial disc implant 100. Also, the articulating interface may be configured such that it ranges from about 0 degrees to about 10 degrees out of alignment with a central axis running from laterally from left to right of the artificial disc implant. In other words, the implant can be configured such that there is angulation of about 0 degrees to about 10 degrees with respect to an axis running laterally from left to right of the implant between mating components of the implant. This misalignment may be beneficial for patients with lordosis. Other aspects of the present invention include specific design features to interact with instrumentation used to manipulate and insert the artificial disc implant.

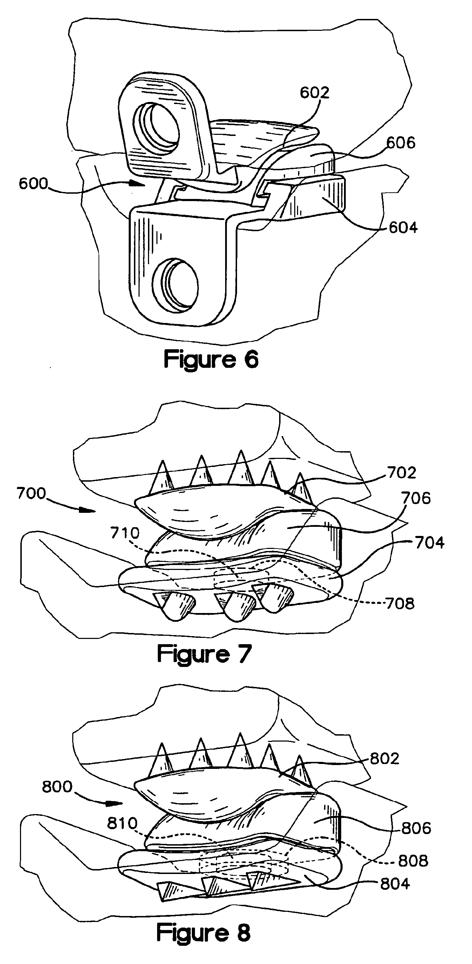

Turning now to Figure 6, an embodiment of the artificial disc implant of the present invention that is configured for screw fixation is provided. The artificial disc implant 600 includes a superior implant 602, an inferior implant 604 and a spacer 606. As shown, the spacer 606 and inferior implant 604 are fixed by a tongue-and-groove fixation method. The embodiment of Figure 6 illustrates one configuration for fixing the superior implant 602 and the inferior implant 604 to the superior vertebral body 6a and the inferior vertebral body 2b respectively by fixation screws. The fixation screws may be made from any material known in art for medical fixation devices. For example, the fixation screws may be made from titanium, titanium/aluminum/vanadium Ti/Al/V alloys, Tantalum, CrCo, carbon or carbon composite materials.

Turning now to Figure 7, an embodiment of the artificial disc implant of the present invention that permits axial rotation is provided. The artificial disc implant 700 includes a superior implant 702, an inferior implant 704 and a spacer 706. The inferior implant 704 includes a cup 708 configured to interact with a disc 710 on the spacer 706. The interaction between the disc 710 and the cup 708 permits axial rotation of the spacer 706 with respect to the inferior implant 704, which permits separation of lateral bending and axial rotation.

Turning now to Figure 8 an embodiment of the artificial disc implant of the present invention that permits generally in-plane motion is provided. The artificial disc implant 800 includes a superior implant 802, an inferior implant 804 and a spacer 806. Like the embodiment of Figure 7, the inferior implant 804 includes a cup 808 configured to interact with a disc 810 on the spacer 806. The interaction between the disc 810 and the cup 808 permits axial rotation of the spacer 806 with respect to the inferior implant 804, which allows for separation of lateral bending and axial rotation. The diameter of the disc 810, however, is substantially smaller than the diameter of the cup 808. In addition to permitting axial rotation, the difference in diameter of the cup 808 and the disc 810 also allows the spacer 806 to achieve generally in-plane motion with respect to the inferior implant 804.

While the present invention has been described in association with several exemplary embodiments, the described embodiments are to be considered in all respects as illustrative and not restrictive. Such other features, aspects, variations, modifications, and substitution of equivalents may be made without departing from the scope of this invention which is intended to be limited solely by the scope of the following claims. Also, it will be appreciated that features and parts illustrated in one embodiment may be used, or may be applicable, in the same or in a similar way in other embodiments.

## Claims

1. An artificial disc implant comprising:
a superior implant (102, 602, 702, 802) configured for placement on a superior vertebra;
an inferior implant (104, 604, 704, 804) configured for placement on an inferior vertebra; and
an articulating interface (116) to be located between the superior vertebra (2a) and the inferior vertebra (2b), the articulating interface (116) being generally saddle-shaped, **characterised in that** the articulating interface is ramped, wherein the articulating interface (116) generally progresses toward the superior vertebra (2a) and away from the inferior vertebra (2b) as the interface progresses from a first side of the artificial disc implant to an opposing side of the artificial disc implant.

2. The artificial disc implant of claim 1 further comprising at least one neutral zone.

3. The artificial disc implant of claim 2 wherein the at least one neutral zone is in at least one of the anterior-posterior direction or the lateral direction.

4. The artificial disc implant of claim 1 wherein the first side of the artificial disc implant is the anterior of the artificial disc implant and the opposing side of the artificial disc implant is the posterior of the artificial disc implant.

5. The artificial disc implant of claim 1 wherein at least one of the superior implant (102, 602, 702, 802) or the inferior implant (104, 604, 704, 804) comprises a fixation surface (108, 112) that is generally flat, generally curved or generally dome-shaped.

6. The artificial disc implant of claim 1 wherein at least one of the superior implant (102, 602, 702, 802) or the inferior implant (104, 604, 704, 804) comprises a fixation mechanism.

7. The artificial disc implant of claim 1 wherein the fixation mechanism comprises at least one of: one or more pegs, one or more fins, one or more pips, ridges, or one or more screws.

8. The artificial disc implant of claim 1 wherein at least one of the superior implant (102, 602, 702, 802) or the inferior implant (104, 604, 704, 804) has a fixation surface comprising at least one of: a porous coating, a porous onlay material, a biologic coating, or a surface treatment.

9. The artificial disc implant of claim 1 wherein the superior implant (102, 602, 702, 802) ranges from about 1 mm thick to about 5 mm thick at the anterior of the artificial disc implant and from about 1 mm to about 5 mm thick at the posterior of the artificial disc implant.

10. The artificial disc implant of claim 1 wherein the articulating interface (116) is configured such that there is angulation of about 0 degrees to about 10 degrees with respect to an axis running laterally from left to right of the implant between mating components of the artificial disc implant.

11. The artificial disc implant of claim 1 wherein the superior implant (102, 602, 702, 802) comprises an articulating surface and the articulating interface is formed by the articulating surface (110) of the superior implant (102, 602, 702, 802) and a second articulating surface (114).

12. The artificial disc implant of claim 11 wherein at least one of the articulating surfaces is composed of at least one of: cobalt-chromium alloy, ceramic, UHMWPE, pyrolytic carbon, titanium with a titanium nitride coating, and Ti/Al/V.

13. The artificial disc implant of claim 11 wherein the second articulating surface is an articulating surface of the inferior implant (104, 604, 704, 804).

14. The artificial disc implant of claim 11 wherein the second articulating surface is an articulating surface (114) of a spacer (106, 606, 706, 806).

15. The artificial disc implant of claim 14 wherein the spacer (106, 606, 706, 806) ranges from about 0.5 mm thick to about 5 mm thick at the anterior of the artificial disc implant and from about 0.5 mm to about 5 mm thick at the posterior of the artificial disc implant.

16. The artificial disc implant of claim 14 wherein the inferior implant (104, 604, 704, 804) ranges from about 0.5 mm thick to about 5 mm thick.

17. The artificial disc implant of claim 14 wherein the spacer (106, 606, 706, 806) is capable of axial rotation with reference to the inferior implant (104, 604, 704, 804).

18. The artificial disc implant of claim 14 wherein the spacer (106, 606, 706, 806) is capable of generally in-plane motion with respect to the inferior implant (104, 604, 704, 804).

19. The artificial disc implant of claim 14 wherein the inferior implant (104, 604, 704, 804) ranges from about 0.5 mm thick to about 5 mm thick at the anterior of the artificial disc implant and from about 0.5 mm to about 5 mm thick at the posterior of the artificial disc implant.

20. The artificial disc implant of claim 1, wherein the superior implant (102, 602, 702, 802) has an articulating surface (110) that is saddle-shaped and ramped such that the articulating surface (110) of the superior implant (102, 602, 702, 802) generally progresses away from superior vertebra (2a) and toward the inferior vertebra (2b) as the articulating surface (110) of the superior implant (102, 602, 702, 802) progresses from the posterior to the anterior of the artificial disc implant; and wherein a spacer (106, 606, 706, 806) is arranged between the superior implant (102, 602, 702, 802) and the inferior implant (104, 604, 704, 804), the spacer (106, 606, 706, 806) having an articulating surface (114) configured to articulate with the articulating surface (110) of the superior implant (102, 602, 702, 802), the articulating surface (114) of the spacer (106, 606, 706, 806) being saddle-shaped and ramped such that the articulating surface (110) of the spacer (106, 606, 706, 806) generally progresses away from the inferior vertebra (2b) and toward the superior vertebra (2a) as the articulating surface (114) of the spacer (106, 606, 706, 806) progresses from the anterior to the posterior of the artificial disc implant.

21. The artificial disc implant of claim 20 further comprising at least one neutral zone.

22. The artificial disc implant of claim 21 wherein the at least one neutral zone is in at least one of the anterior-posterior direction or the lateral direction.

23. The artificial disc implant of claim 20 wherein the spacer (106, 606, 706, 806) is fixed to the inferior implant (104, 604, 704, 804).

24. The artificial disc implant of claim 20 wherein at least one of the superior implant (102, 602, 702, 802) or the inferior implant (104, 604, 704, 804) comprises a fixation mechanism.

25. The artificial disc implant of claim 20 wherein the fixation mechanism comprises at least one of: one or more pegs, one or more fins, one or more pips, ridges, or one or more screws.

26. The artificial disc implant of claim 20 wherein at least one of the superior implant (102, 602, 702, 802) or the inferior implant (102, 602, 702, 802) has a fixation surface (108, 112) comprising at least one of: a porous coating, a porous onlay material, a biologic coating, or a surface treatment.

27. The artificial disc implant of claim 20 wherein at least one of the articulating surfaces (110, 114) of the superior implant (102, 602, 702, 802) and the spacer (106, 606, 706, 806) is composed of at least one of: cobalt-chromium alloy, ceramic, UHMWPE, pyrolytic carbon, titanium with a titanium nitride coating, or Ti/Al/V.

28. The artificial disc implant of claim 20 wherein the spacer (106, 606, 706, 806) is capable of axial rotation with reference to the inferior implant (104, 604, 704, 804).

29. The artificial disc implant of claim 25 wherein the spacer (106, 606, 706, 806) is capable of translation with respect to the inferior implant (104, 604, 704, 804).

30. The artificial disc implant of claim 1 wherein the superior implant (102, 602, 702, 802) has a fixation surface (108) configured for placement on a superior vertebra (2a) and an articulating surface (110) that is saddle-shaped and ramped such that the articulating surface of the superior implant (102, 602, 702, 802) generally progresses away from the superior vertebra (2a) and toward the inferior vertebra (2b) as the articulating surface (110) of the superior implant (102, 602, 702, 802) progresses from the posterior to the anterior of the artificial disc implant; and
wherein the inferior implant (104, 604, 704, 804) has a fixation surface configured for placement on an inferior vertebra (2b) and an articulating surface configured to articulate with the articulating surface of the superior implant, the articulating surface of the inferior implant (104, 604, 704, 804) being saddle-shaped and ramped such that the articulating surface of the inferior implant generally progresses away from the inferior vertebra and toward the superior vertebra as the articulating surface of the inferior implant progresses from the anterior to the posterior of the artificial disc implant.

31. The artificial disc implant of claim 30 further comprising at least one neutral zone.

32. The artificial disc implant of claim 31 wherein the at least one neutral zone is in at least one of the anterior-posterior direction or the lateral direction.

## Patentansprüche

1. Künstliches Bandscheibenimplantat mit:
einem oberen Implantat (102,602,702,802), das zur Platzierung auf einem oberen Rückenwirbel konfiguriert ist;
einem unteren Implantat (104,604,704,804), das zur Platzierung auf einem unteren Rückenwirbel konfiguriert ist; und
einem Artikulations-Interface (116) zur Anordnung zwischen dem oberen Rückenwirbel (2a) und dem unteren Rückenwirbel (2b), wobei das Artikulations-Interface (116) im Wesentlichen sattelförmig ist,
**dadurch gekennzeichnet, dass** das Artikulations-Interface im Wesentlichen rampenförmig ist, wobei im Verlauf des Interface von einer ersten Seite des künstlichen Bandscheibenimplantats zu der gegenüberliegenden Seite des künstlichen Bandscheibenimplantats das Artikulations-Interface (116) im Wesentlichen zu dem oberen Rückenwirbel (2a) hin und von dem unteren Rückenwirbel (2b) weg verläuft.

2. Künstliches Bandscheibenimplantat nach Anspruch 1, ferner mit mindestens einer neutralen Zone.

3. Künstliches Bandscheibenimplantat nach Anspruch 2, bei dem die mindestens eine neutrale Zone mindestens in der von einem Vorderbereich zu einem Rückbereich verlaufenden Richtung oder der Seitenrichtung gelegen ist.

4. Künstliches Bandscheibenimplantat nach Anspruch 1, bei dem die erste Seite des künstlichen Bandscheibenimplantats der Vorderbereich des künstlichen Bandscheibenimplantats und die gegenüberliegende Seite des künstlichen Bandscheibenimplantats der Rückbereich des künstlichen Bandscheibenimplantats ist.

5. Künstliches Bandscheibenimplantat nach Anspruch 1, bei dem mindestens das obere Implantat (102,602,702,802) oder das untere Implantat (104,604,704,804) eine Fixierfläche (108,112) aufweist, die im Wesentlichen flach, im Wesentlichen gekrümmt oder im Wesentlichen domförmig ist.

6. Künstliches Bandscheibenimplantat nach Anspruch 1, bei dem mindestens das obere Implantat (102,602,702,802) oder das untere Implantat (104,604,704,804) einen Fixiermechanismus aufweist.

7. Künstliches Bandscheibenimplantat nach Anspruch 1, bei dem der Fixiermechanismus mindestens eines der folgenden Elemente aufweist:
einen oder mehrere Stifte, eine oder mehrere Rippen, eines oder mehrere Zacken, Grate, oder eine oder mehrere Schrauben.

8. Künstliches Bandscheibenimplantat nach Anspruch 1, bei dem mindestens das obere Implantat (102,602,702,802) oder das untere Implantat (104,604,704,804) eine Fixierfläche mit mindestens einem der folgenden Elemente aufweist: eine poröse Beschichtung, ein poröses Auflagematerial, eine biologische Beschichtung oder eine Oberflächenbehandlung.

9. Künstliches Bandscheibenimplantat nach Anspruch 1, bei dem das obere Implantat (102,602,702,802) eine Dicke im Bereich von ungefähr 1 mm bis ungefähr 5 mm am Vorderbereich des künstlichen Bandscheibenimplantats und im Bereich von ungefähr 1 mm bis ungefähr 5 mm am Rückbereich des künstlichen Bandscheibenimplantats hat.

10. Künstliches Bandscheibenimplantat nach Anspruch 1, bei dem das Artikulations-Interface (116) derart konfiguriert ist, dass eine Winkligkeit von ungefähr 0 Grad bis ungefähr 10 Grad relativ zu einer Achse besteht, die seitlich von der linken zu der rechten Seite des Implantats zwischen passend zusammengreifenden Bestandteilen des künstlichen Bandscheibenimplantats verläuft.

11. Künstliches Bandscheibenimplantat nach Anspruch 1, bei dem das obere Implantat (102,602,702,802) ein Artikulations-Interface aufweist und das Artikulations-Interface durch die Artikulationsfläche (110) des oberen Implantats (102,602,702,802) und eine zweite Artikulationsfläche (114) gebildet ist.

12. Künstliches Bandscheibenimplantat nach Anspruch 11, bei dem mindestens eine der Artikulationsflächen aus mindestens einem der folgenden Elemente gebildet ist: einer Kobalt-Chrom-Legierung, Keramik, UHMWPE, pyrolitischem Kohlenstoff, Titan mit einer Titannitrid-Beschichtung, und Ti/Al/V.

13. Künstliches Bandscheibenimplantat nach Anspruch 11, bei dem die zweite Artikulationsfläche eine Artikulationsfläche des unteren Implantats (104,604,704,804) ist.

14. Künstliches Bandscheibenimplantat nach Anspruch 11, bei dem die zweite Artikulationsfläche eine Artikulationsfläche (114) eines Abstandhalters (106,606,706,806) ist.

15. Künstliches Bandscheibenimplantat nach Anspruch 14, bei dem der Abstandhalter (106,606,706,806) eine Dicke im Bereich von ungefähr 0,5 mm bis ungefähr 5 mm am Vorderbereich des künstlichen Bandscheibenimplantats und im Bereich von ungefähr 0,5 mm bis ungefähr 5 mm am Rückbereich des künstlichen Bandscheibenimplantats hat.

16. Künstliches Bandscheibenimplantat nach Anspruch 14, bei dem das untere Implantat (104,604,704,804) eine Dicke im Bereich von ungefähr 0,5 mm bis ungefähr 5 mm hat.

17. Künstliches Bandscheibenimplantat nach Anspruch 14, bei dem der Abstandhalter (106,606,706,806) relativ zu dem unteren Implantat (104,604,704,804) axial drehbar ist.

18. Künstliches Bandscheibenimplantat nach Anspruch 14, bei dem der Abstandhalter (106,606,706,806) relativ zu dem unteren Implantat (104,604,704,804) im Wesentlichen in der gleichen Ebene bewegbar ist.

19. Künstliches Bandscheibenimplantat nach Anspruch 14, bei dem das untere Implantat (104,604,704,804) eine Dicke im Bereich von ungefähr 0,5 mm bis ungefähr 5 mm am Vorderbereich des künstlichen Bandscheibenimplantats und im Bereich von ungefähr 0,5 mm bis ungefähr 5 mm am Rückbereich des künstlichen Bandscheibenimplantats hat.

20. Künstliches Bandscheibenimplantat nach Anspruch 1, bei dem das obere Implantat (102,602,702,802) eine Artikulationsfläche (110) aufweist, die derart sattelförmig und rampenförmig ist, dass im Verlauf der Artikulationsfläche (110) des oberen Implantats (102,602,702, 802) von dem Vorderbereich zu dem Rückbereich des künstlichen Bandscheibenimplantats die Artikulationsfläche (110) des oberen Implantats (102,602,702,802) im Wesentlichen von dem oberen Rückenwirbel (2a) weg und zu dem unteren Rückenwirbel (2b) hin verläuft; und bei dem ein Abstandhalter (106,606,706,806) zwischen dem oberen Implantat (102,602,702,802) und dem unteren Implantat (104,604,704,804) angeordnet ist, wobei der Abstandhalter (106,606, 706,806) eine Artikulationsfläche (114) aufweist, die zur Artikulation mit der Artikulationsfläche (110) des oberen Implantats (102,602,702, 802) konfiguriert ist, wobei die Artikulationsfläche (114) des Abstandhalters (106,606,706,806) derart sattelförmig und rampenförmig ist, dass im Verlauf der Artikulationsfläche (114) des Abstandhalters (106, 606,706,806) von dem Vorderbereich zu dem Rückbereich des künstlichen Bandscheibenimplantats die Artikulationsfläche (110) des Abstandhalters (106,606,706,806) im Wesentlichen von dem unteren Rückenwirbel (2b) weg und zu dem oberen Rückenwirbel (2a) hin verläuft.

21. Künstliches Bandscheibenimplantat nach Anspruch 20, ferner mit mindestens einer neutralen Zone.

22. Künstliches Bandscheibenimplantat nach Anspruch 21, bei dem die mindestens eine neutrale Zone mindestens in der von dem Vorderbereich zu dem Rückbereich verlaufenden Richtung oder der Seitenrichtung gelegen ist.

23. Künstliches Bandscheibenimplantat nach Anspruch 20, bei dem der Abstandhalter (106,606,706,806) an dem unteren Implantat (104,604, 704,804) fixiert ist.

24. Künstliches Bandscheibenimplantat nach Anspruch 20, bei dem mindestens das obere Implantat (102,602,702,802) oder das untere Implantat (104,604,704,804) einen Fixiermechanismus aufweist.

25. Künstliches Bandscheibenimplantat nach Anspruch 20, bei dem der Fixiermechanismus mindestens eines der folgenden Elemente aufweist:
einen oder mehrere Stifte, eine oder mehrere Rippen, eines oder mehrere Zacken, Grate, oder eine oder mehrere Schrauben.

26. Künstliches Bandscheibenimplantat nach Anspruch 20, bei dem mindestens das obere Implantat (102,602,702,802) oder das untere Implantat (104,604,704,804) eine Fixierfläche (108,112) mit mindestens einem der folgenden Elemente aufweist: eine poröse Beschichtung, ein poröses Auflagematerial, eine biologische Beschichtung oder eine Oberflächenbehandlung.

27. Künstliches Bandscheibenimplantat nach Anspruch 20, bei dem mindestens eine der Artikulationsflächen (110,114) des oberen Implantats (102,602,702,802) und des Abstandhalters (106,606,706,806) aus mindestens einem der folgenden Elemente gebildet ist: einer Kobalt-Chrom-Legierung, Keramik, UHMWPE, pyrolitischem Kohlenstoff, Titan mit einer Titannitrid-Beschichtung, oder Ti/Al/V.

28. Künstliches Bandscheibenimplantat nach Anspruch 20, bei dem der Abstandhalter (106,606,706,806) relativ zu dem unteren Implantat (104,604,704,804) axial drehbar ist.

29. Künstliches Bandscheibenimplantat nach Anspruch 25, bei dem der Abstandhalter (106,606,706,806) relativ zu dem unteren Implantat (104,604,704,804) translatorisch bewegbar ist.

30. Künstliches Bandscheibenimplantat nach Anspruch 1, bei dem das obere Implantat (102,602,702,802) eine Fixierfläche (108), die zur Platzierung auf einem oberen Rückenwirbel (2a) konfiguriert ist, und eine Artikulationsfläche (110) aufweist, die derart sattelförmig und rampenförmig ist, dass im Verlauf der Artikulationsfläche (110) des oberen Implantats (102,602,702,802) von dem Vorderbereich zu dem Rückbereich des künstlichen Bandscheibenimplantats die Artikulationsfläche des oberen Implantats (102,602,702,802) im Wesentlichen von dem oberen Rückenwirbel (2a) weg und zu dem unteren Rückenwirbel (2b) hin verläuft; und bei dem das untere Implantat (104,604,704,804) eine Fixierfläche, die zur Platzierung auf einem unteren Rückenwirbel (2b) konfiguriert ist, und eine Artikulationsfläche aufweist, die zur Artikulation mit der Artikulationsfläche des oberen Implantats konfiguriert ist, wobei die Artikulationsfläche des unteren Implantats (104,604,704, 804) derart sattelförmig und rampenförmig ist, dass im Verlauf der Artikulationsfläche des unteren Implantats von dem Vorderbereich zu dem Rückbereich des künstlichen Bandscheibenimplantats die Artikulationsfläche des unteren Implantats von dem unteren Rückenwirbel weg und zu dem oberen Rückenwirbel hin verläuft.

31. Künstliches Bandscheibenimplantat nach Anspruch 30, ferner mit mindestens einer neutralen Zone.

32. Künstliches Bandscheibenimplantat nach Anspruch 31, bei dem die mindestens eine neutrale Zone mindestens in der von einem Vorderbereich zu einem Rückbereich verlaufenden Richtung oder der Seitenrichtung gelegen ist.

## Revendications

1. Implant de disque artificiel comprenant :
un implant supérieur (102, 602, 702, 802) configuré pour être placé sur une vertèbre supérieure ;
un implant inférieur (104, 604, 704, 804) configuré pour être placé sur une vertèbre inférieure ; et
une interface d'articulation (116) devant être placée entre la vertèbre supérieure (2a) et la vertèbre inférieure (2b), l'interface d'articulation (116) étant généralement en forme de selle, **caractérisé en ce que** l'interface d'articulation est inclinée, l'interface d'articulation (116), généralement, avançant vers la vertèbre supérieure (2a) et s'éloignant de la vertèbre inférieure (2b) à mesure que l'interface avance d'un premier côté de l'implant de disque artificiel vers un côté opposé de l'implant de disque artificiel.

2. Implant de disque artificiel selon la revendication 1, comprenant en outre au moins une zone neutre.

3. Implant de disque artificiel selon la revendication 2, dans lequel la ou les zones neutres sont dans au moins une des directions antéro-postérieure ou latérale.

4. Implant de disque artificiel selon la revendication 1, dans lequel le premier côté de l'implant de disque artificiel est la partie antérieure de l'implant de disque artificiel et le côté opposé de l'implant de disque artificiel est la partie postérieure de l'implant de disque artificiel.

5. Implant de disque artificiel selon la revendication 1, dans lequel au moins un élément parmi l'implant supérieur (102, 602, 702, 802) et l'implant inférieur (104, 604, 704, 804) comprend une surface de fixation (108, 112) qui est généralement plate, généralement incurvée, ou généralement en forme de dôme.

6. Implant de disque artificiel selon la revendication 1, dans lequel au moins un élément parmi l'implant supérieur (102, 602, 702, 802) et l'implant inférieur (104, 604, 704, 804) comprend un mécanisme de fixation.

7. Implant de disque artificiel selon la revendication 1, dans lequel le mécanisme de fixation comprend au moins un élément parmi : une ou plusieurs chevilles, une ou plusieurs ailettes, une ou plusieurs pointes, des arêtes, ou une ou plusieurs vis.

8. Implant de disque artificiel selon la revendication 1, dans lequel au moins un élément parmi l'implant supérieur (102, 602, 702, 802) et l'implant inférieur (104, 604, 704, 804) a une surface de fixation comprenant au moins un élément parmi : un revêtement poreux, un matériau de greffe apposée poreux, un revêtement biologique, ou un traitement de surface.

9. Implant de disque artificiel selon la revendication 1, dans lequel l'implant supérieur (102, 602, 702, 802) a une épaisseur dans la plage d'environ 1 mm à environ 5 mm au niveau de la partie antérieure de l'implant de disque artificiel et d'environ 1 mm à environ 5 mm au niveau de la partie postérieure de l'implant de disque artificiel.

10. Implant de disque artificiel selon la revendication 1, dans lequel l'interface d'articulation (116) est configurée de manière à ce qu'il y ait une angulation d'environ 0 degrés à environ 10 degrés par rapport à un axe s'étendant latéralement de gauche à droite de l'implant entre les composants accouplés de l'implant de disque artificiel.

11. Implant de disque artificiel selon la revendication 1, dans lequel l'implant supérieur (102, 602, 702, 802) comprend une surface d'articulation et l'interface d'articulation est formée par la surface d'articulation (110) de l'implant supérieur (102, 602, 702, 802) et une seconde surface d'articulation (114).

12. Implant de disque artificiel selon la revendication 11, dans lequel au moins une des surfaces d'articulation est composée d'un moins un élément parmi : l'alliage cobalt-chrome, la céramique, l'UHMWPE, le pyrocarbone, le titane avec un revêtement de nitrure de titane, et le Ti/Al/V.

13. Implant de disque artificiel selon la revendication 11, dans lequel la seconde surface d'articulation est une surface d'articulation de l'implant inférieur (104, 604, 704, 804).

14. Implant de disque artificiel selon la revendication 11, dans lequel la seconde surface d'articulation est une surface d'articulation (114) d'une pièce d'écartement (106, 606, 706, 806).

15. Implant de disque artificiel selon la revendication 14, dans lequel la pièce d'écartement (106, 606, 706, 806) a une épaisseur dans la plage d'environ 0,5 mm à environ 5 mm au niveau de la partie antérieure de l'implant de disque artificiel et d'environ 0,5 mm à environ 5 mm au niveau de la partie postérieure de l'implant de disque artificiel.

16. Implant de disque artificiel selon la revendication 14, dans lequel l'implant inférieur (104, 604, 704, 804) a une épaisseur dans la plage d'environ 0,5 mm à environ 5 mm.

17. Implant de disque artificiel selon la revendication 14, dans lequel la pièce d'écartement (106, 606, 706, 806) peut effectuer une rotation axiale par rapport à l'implant inférieur (104, 604, 704, 804).

18. Implant de disque artificiel selon la revendication 14, dans lequel la pièce d'écartement (106, 606, 706, 806) peut effectuer un mouvement généralement dans le plan par rapport à l'implant inférieur (104, 604, 704, 804).

19. Implant de disque artificiel selon la revendication 14, dans lequel l'implant inférieur (104, 604, 704, 804) a une épaisseur dans la plage d'environ 0,5 mm à environ 5 mm au niveau de la partie antérieure de l'implant de disque artificiel et d'environ 0,5 mm à environ 5 mm au niveau de la partie postérieure de l'implant de disque artificiel.

20. Implant de disque artificiel selon la revendication 1, dans lequel
l'implant supérieur (102, 602, 702, 802) a une surface d'articulation (110) qui est en forme de selle et inclinée de telle sorte que la surface d'articulation (110) de l'implant supérieur (102, 602, 702, 802) s'éloigne généralement de la vertèbre supérieure (2a) et avance vers la vertèbre inférieure (2b) à mesure que la surface d'articulation (110) de l'implant supérieur (102, 602, 702, 802) avance de la partie postérieure vers la partie antérieure de l'implant de disque artificiel ; et dans lequel une pièce d'écartement (106, 606, 706, 806) est agencée entre l'implant supérieur (102, 602, 702, 802) et l'implant inférieur (104, 604, 704, 804), la pièce d'écartement (106, 606, 706, 806) ayant une surface d'articulation (114) configurée pour s'articuler avec la surface d'articulation (110) de l'implant supérieur (102, 602, 702, 802), la surface d'articulation (114) de la pièce d'écartement (106, 606, 706, 806) étant en forme de selle et inclinée de telle sorte que la surface d'articulation (110) de la pièce d'écartement (106, 606, 706, 806) s'éloigne généralement de la vertèbre inférieure (2b) et avance vers la vertèbre supérieure (2a) à mesure que la surface d'articulation (114) de la pièce d'écartement (106, 606, 706, 806) avance de la partie antérieure vers la partie postérieure de l'implant de disque artificiel.

21. Implant de disque artificiel selon la revendication 20, comprenant en outre au moins une zone neutre.

22. Implant de disque artificiel selon la revendication 21, dans lequel la ou les zones neutres sont dans au moins une des directions antéro-postérieure ou latérale.

23. Implant de disque artificiel selon la revendication 20, dans lequel la pièce d'écartement (106, 606, 706, 806) est fixée à l'implant inférieur (104, 604, 704, 804).

24. Implant de disque artificiel selon la revendication 20, dans lequel au moins un élément parmi l'implant supérieur (102, 602, 702, 802) et l'implant inférieur (104, 604, 704, 804) comprend un mécanisme de fixation.

25. Implant de disque artificiel selon la revendication 20, dans lequel le mécanisme de fixation comprend au moins un élément parmi : une ou plusieurs chevilles, une ou plusieurs ailettes, une ou plusieurs pointes, des arêtes, ou une ou plusieurs vis.

26. Implant de disque artificiel selon la revendication 20, dans lequel au moins un élément parmi l'implant supérieur (102, 602, 702, 802) et l'implant inférieur (104, 604, 704, 804) a une surface de fixation (108, 112) comprenant au moins un élément parmi : un revêtement poreux, un matériau de greffe apposée poreux, un revêtement biologique, ou un traitement de surface.

27. Implant de disque artificiel selon la revendication 20, dans lequel au moins une des surfaces d'articulation (110, 114) de l'implant supérieur (102, 602, 702, 802) et de la pièce d'écartement (106, 606, 706, 806) est composée d'un moins un élément parmi : l'alliage cobalt-chrome, la céramique, l'UHMWPE, le pyrocarbone, le titane avec un revêtement de nitrure de titane, et le Ti/Al/V.

28. Implant de disque artificiel selon la revendication 20, dans lequel la pièce d'écartement (106, 606, 706, 806) peut effectuer une rotation axiale par rapport à l'implant inférieur (104, 604, 704, 804).

29. Implant de disque artificiel selon la revendication 25, dans lequel la pièce d'écartement (106, 606, 706, 806) peut effectuer une translation par rapport à l'implant inférieur (104, 604, 704, 804).

30. Implant de disque artificiel selon la revendication 1, dans lequel l'implant supérieur (102, 602, 702, 802) a une surface de fixation (108) configurée pour être placée sur une vertèbre supérieure (2a) et une surface d'articulation (110) qui est en forme de selle et inclinée de telle sorte que la surface d'articulation de l'implant supérieur (102, 602, 702, 802) s'éloigne généralement de la vertèbre supérieure (2a) et avance vers la vertèbre inférieure (2b) à mesure que la surface d'articulation (110) de l'implant supérieur (102, 602, 702, 802) avance de la partie postérieure vers la partie antérieure de l'implant de disque artificiel ; et
dans lequel l'implant inférieur (104, 604, 704, 804) a une surface de fixation configurée pour être placée sur une vertèbre inférieure (2b) et une surface d'articulation configurée pour s'articuler avec la surface d'articulation de l'implant supérieur, la surface d'articulation de l'implant inférieur (104, 604, 704, 804) étant en forme de selle et inclinée de telle sorte que la surface d'articulation de l'implant inférieur s'éloigne généralement de la vertèbre inférieure et avance vers la vertèbre supérieure à mesure que la surface d'articulation de l'implant inférieur avance de la partie antérieure vers la partie postérieure de l'implant de disque artificiel.

31. Implant de disque artificiel selon la revendication 30, comprenant en outre au moins une zone neutre.

32. Implant de disque artificiel selon la revendication 31, dans lequel la ou les zones neutres sont dans au moins une des directions antéro-postérieure ou latérale.
